# EUROPÄISCHE PATENTANMELDUNG

(11) **EP 2 987 609 A1**
(43) Veröffentlichungstag der Anmeldung: **24.02.2016**
(21) Anmeldenummer: 14181655.3
(22) Anmeldetag: 20.08.2014
(51) Int. Cl.: B29C 49/46

(54) **Formfüllmaschine mit Reinigung und Verfahren**

(71) Anmelder: Krones AG, 93073 Neutraubling (DE)
(72) Erfinder: Frankenberger, Guenter, 93073 Neutraubling (DE)
(74) Vertreter: Grünecker Patent- und Rechtsanwälte PartG mbB

(57) **Zusammenfassung**

Formfüllanlage (1) mit einer Formfüllmaschine (5) zum Formen von Vorformlingen zu Behältern und Füllen der Behälter in einer oder mehreren Formfüllstationen und mit einem stromaufwärts der Formfüllmaschine angeordneten Heizmodul (7), in dem Vorformlinge aufgeheizt werden können, dadurch gekennzeichnet, dass in Transportrichtung der Vorformlinge nach dem Heizmodul eine Reinigungseinrichtung angeordnet ist, die den Vorformling reinigen kann und ein Verfahren zum Herstellen von Behältern aus Vorformlingen.

## Beschreibung

Die Erfindung betrifft eine Formfüllmaschine und ein Verfahren zum Ausformen und Füllen von Kunststoffbehältern.

### Stand der Technik

Bekanntermaßen lassen sich Kunststoffbehälter im Streckblasverfahren aus Vorformlingen herstellen.

Alternativ zu einem Aufblasen der Behälter mit Pressluft beschreibt die EP 1529620 B1 ein Verfahren zum hydraulischen Umformen von Vorformlingen zu Kunststoffflaschen. Zu diesem Zweck werden die Vorformlinge zuerst erwärmt, in eine Hohlform gebracht und dort in Längsrichtung gereckt. Es wird ferner ein Fluid eingeleitet, um die endgültige Behälterform herzustellen. Das Fluid enspricht der im Behälter zu verbleibenden Flüssigkeit und verbleibt somit im Behälter, sodass ein nachfolgender separater Abfüllschritt entbehrlich ist.

Die US 2011/0031659 A1 beschreibt ferner ein Verfahren, bei dem ein erwärmter Vorformling mittels einer Reckstange gereckt und anschließend mittels eines inkompressiblen Fluids, insbesondere Wasser, hydraulisch zu einem Behälter geweitet wird. Danach wird das Fluid durch Pressluft verdrängt und läuft aus dem Behälter ab.

Eine Formfüllmaschine umfasst definitionsgemäß wenigstens eine Behandlungsstation zum expandierenden Umformen von Kunststoffvorformlingen zu Kunststoffbehältern in einer Hohlform und zum Einfüllen eines im Wesentlichen flüssigen Produkts oder wenigstens einer flüssigen oder festen Komponente des Produkts in die Kunststoffbehälter.

Flüssigkeiten, auch solche mit darin gelöstem Kohlendioxid oder dergleichen, sind definitionsgemäß hinsichtlich ihrer Funktion beim Ausformen und Füllen der Behälter inkompressible Fluide im Gegensatz zu Gasen, die funktional als kompressible Fluide definiert sind.

### Aufgabe

Ausgehend vom Stand der Technik ist es Aufgabe der Erfindung, eine Formfüllanlage bereitzustellen, mit der ein Befüllen eines in der Formfüllanlage ausgeformten sauberen, vorzugsweise sogar sterilisierten Behälters ermöglicht wird.

### Lösung

Diese Aufgabe wird durch die Formfüllanlage nach Anspruch 1 und das Verfahren zum Herstellen von Behältern aus Vorformlingen nach Anspruch 9 gelöst. Vorteilhafte Weiterbildungen der Erfindung sind in den Unteransprüchen erfasst.

Die erfindungsgemäße Formfüllanlage ist dadurch gekennzeichnet, dass in Transportrichtung der Vorformlinge nach dem Heizmodul eine Reinigungseinrichtung angeordnet ist, die den Vorformling reinigen kann. Durch die dem Aufheizen nachgeordnete Reinigung kann der Vorformling vor dem Ausformen gereinigt, vorzugsweise sogar sterilisiert werden, so dass beim Ausformen und Füllen des Behälters ein möglichst sauberer Behälter vorliegt.

In einer Ausführungsform ist die Formfüllanlage dadurch gekennzeichnet, dass die Reinigungseinrichtung ein gasförmiges oder flüssiges Reinigungsmedium in den Vorformling einbringen kann. In einer Ausführungsform kann das Reinigungsmedium eine sterilisierende Wirkung aufweisen, etwa als sterilisierendes Gas.

In einer weiteren Ausführungsform ist die Reinigungseinrichtung an einer Transportstrecke vor der Formfüllmaschine angeordnet. Die Reinigung des Vorformlings mit der Reinigungseinrichtung vor dem Einlauf in die Formfüllmaschine erlaubt eine Entkopplung der Reinigung von den übrigen Prozessschritten, so dass beispielsweise ein der Reinigung nachfolgendes Inspizieren ein Aussortieren nicht korrekt gereinigter Vorformling ermöglichen kann. Zusätzlich ermöglicht diese Anordnungsform gegebenenfalls auch die Möglichkeit eines Aufschwenkens der Preform während der Reinigung, um das Austragen von Verschmutzungen zu erleichtern.

In einer anderen Ausführungsform ist die Reinigungseinrichtung in der Formfüllmaschine angeordnet und kann den Vorformling vor dem Ausformen reinigen. Diese Ausführungsform erlaubt das Bereitstellen einer kompakten Formfüllanlage, da hier Reinigung, Ausformung und Befüllen in einer Maschine erfolgen kann. Weiterhin wird so das Risiko von Kontamination nach dem Reinigen reduziert.

In einer Weiterbildung dieser Ausführungsform ist ein in den Vorformling einführbares Reinigungselement in jeder Formfüllstation vorgesehen, das ein Reinigungsmedium in den Vorformling einbringen kann. Das Reinigungselement kann beispielsweise in Form einer Lanze in den Vorformling eingeführt werden, sodass ein direkter Eintrag von Reinigungsmedium in den Vorformling möglich ist.

In einer weiteren Weiterbildung ist die Formfüllanlage dadurch gekennzeichnet, dass ein

Behandlungsstutzen (oder alternativ: "ein kombinierter Füll- und Behandlungsstutzen") in der Formfüllstation vorgesehen ist, der ein Reinigungsventil und ein Formventil umfasst, wobei der Behandlungsstutzen zur Reinigung des Vorformlings in diesen eingeführt werden kann und den Vorformling luftdicht abschließen kann und wobei durch das Formventil ein Medium in den gereinigten und abgeschlossenen Vorformling zum Ausformen des Behälters eingebracht werden kann. Das luftdichte Abschließen des Vorformlings durch den Behandlungsstutzen stellt sicher, dass zumindest die Innenseite des Vorformlings nach der Reinigung nicht weiter kontaminiert wird.

Das definierte Abführen des Spülgases durch den Stutzen ermöglicht reproduzierbare Reinigungsvorgänge und verhindert eine Querkontamination der Nebenstationen mit ausgespültem Gas.

In einer vorteilhaften Weiterbildung ist das Formventil ausgebildet, Produkt in den Behälter einzufüllen. Da so direkt nach der Reinigung Produkt in den Behälter eingeleitet werden kann, wird die Zeit zwischen Reinigung und Befüllung reduziert, was das Risiko unerwünschter Kontaminationen weiter verringert.

Ferner kann das Formventil ausgebildet sein, das Ausformen des Behälters direkt mit dem Produkt als Medium durchzuführen. Somit wird die Zeit zwischen Reinigung und Befüllen noch weiter reduziert und das Risiko von Kontamination minimiert.

Das erfindungsgemäße Verfahren zum Herstellen von Behältern aus Vorformlingen ist dadurch gekennzeichnet, dass die Vorformlinge vor dem Ausformen gereinigt werden. Mit diesem Verfahren können saubere Vorformlinge für das Formfüllen bereitgestellt werden, womit das Risiko für eine Kontamination des Produkts, das in den Behälter geleitet wird, reduziert wird.

In einer Ausführungsform ist das Verfahren dadurch gekennzeichnet, dass die Vorformlinge mit einem flüssigen oder einem gasförmigen Medium gereinigt werden.

Vorteilhaft ist, wenn die Vorformlinge vor Einlauf in die Formfüllmaschine gereinigt werden. Diese Ausführungsform erlaubt beispielsweise eine Inspektion nach dem Reinigen, so dass nicht ordnungsgemäß gereinigte Vorformlinge gegebenenfalls aussortiert werden können.

Alternativ können die Vorformlinge innerhalb der Formfüllmaschine gereinigt werden. Somit kann die Zeit zwischen Reinigen und anschließendem Formfüllen reduziert werden, was grundsätzlich das Risiko für Kontamination verringert.

In einer Weiterbildung dieser Ausführungsform ist vorgesehen, dass die Vorformlinge während der Reinigung und dem Ausformen kontinuierlich luftdicht verschlossen sind. Durch das Verschließen wird das Kontaminationsrisiko weiter verringert.

Auch vorteilhaft ist, wenn die Vorformlinge befüllt werden, während sie luftdicht verschlossen sind. Das Abfüllen des Produkts kann so unter kontrollierter Atmosphäre erfolgen.

Es kann ferner vorgesehen sein, dass das zum Ausformen verwendete Medium das in den Behälter einzufüllende Produkt ist. Durch die reduzierte Zeit zwischen der Reinigung und dem Befüllen während des Ausformens kann so das Risiko unerwünschter Kontaminationen noch weiter verringert werden.

**Kurze Beschreibung der Figuren**
- Fig. 1: Schematische Darstellung einer Formfüllanlage gemäß einer Ausführungsform.
- Fig. 2a+2b: Schematische Darstellung zweier Ausführungsformen der Reinigungsvorrichtung in der Formfüllanlage.
- Fig. 3: Schematische Darstellung einer Ausführungsform einer Reinigungsanlage außerhalb der Formfüllanlage.
- Fig. 4a-f: Schematische Darstellung eines Verfahrens zum Reinigen eines Vorformlings in der Formfüllmaschine gemäß einer Ausführungsform.

### Ausführliche Beschreibung

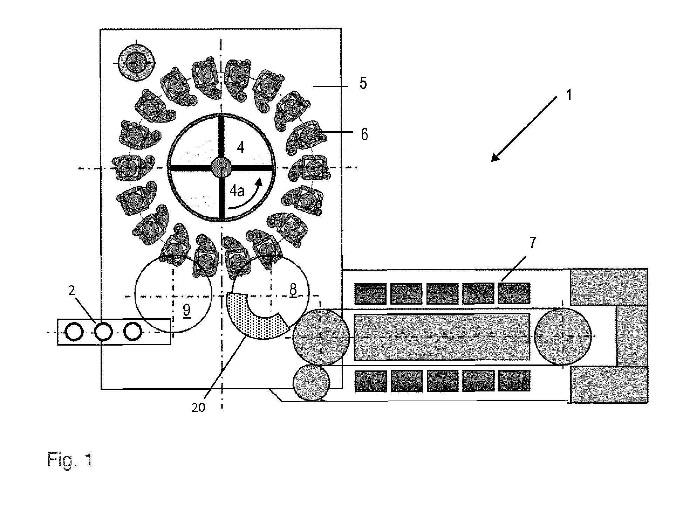

In Fig. 1 ist eine erfindungsgemäße Formfüllanlage 1 dargestellt. Diese Formfüllanlage kann genutzt werden, um Vorformlinge beispielsweise aus PET oder anderen Kunststoffen aufzuheizen und dann zu fertigen Behältern auszuformen und zu befüllen. Dazu verfügt die Formfüllanlage über ein Heizmodul 7, in dem die diesem Heizmodul 7 zugeführten Vorformlinge aufgeheizt werden können.

Dabei kann das Heizmodul beispielsweise ein Ofen sein, der die Vorformlinge über entsprechende Wärmestrahlung aufheizt. Weiterhin können hierzu auch Infrarotquellen oder andere Strahlungsquellen, sowie warme Luft verwendet werden.

Von dem Heizmodul aus wird der Vorformling durch entsprechende Transportmittel 8, wie beispielsweise Drehsterne oder Förderbänder, weitertransportiert und gelangt zur Formfüllmaschine 5. Sie kann als Rundläufermaschine mit einem in Richtung 4a drehbaren Karussell 4 ausgebildet sein, an dem mehrere Formfüllstationen 6 angeordnet sind, in denen ein Vorformling zu einem Behälter ausgeformt und befüllt werden kann. Dabei kann das Ausformen und das Befüllen im selben Behandlungsschritt erfolgen oder zeitlich nacheinander erfolgen, so dass zunächst der Behälter ausgeformt (beispielsweise durch Blasformverfahren und/oder durch Strecken oder durch Hineinpressen einer nicht kompressiblen Flüssigkeit) und anschließend in der Formfüllmaschine 6 befüllt wird. Dabei erfolgen Ausformen und Befüllen in derselben Formfüllstation. Der ausgeformte und befüllte Behälter 2 kann dann über entsprechende weitere Transportmittel 9 abtransportiert werden.

Um sicherzustellen, dass der Vorformling und insbesondere seine Innenfläche sauber, möglichst sogar steril ist, bevor das Produkt eingefüllt wird, ist erfindungsgemäß vorgesehen, dass stromabwärts des Heizmoduls eine Reinigungseinrichtung 20 angeordnet ist, die den Vorformling vor dem Ausformen reinigen kann. Dabei ist insbesondere vorgesehen, dass die Innenseite des Vorformlings, also die Fläche, die nach dem Ausformen mit dem Produkt in Kontakt kommt, möglichst sauber, vorzugsweise sogar sterilisiert ist.

Dabei kann die Reinigungseinrichtung 20 entweder, wie dargestellt, als vor der Formfüllmaschine 5 aber im Bereich der Transporteinrichtung 8 ausgebildet oder in die Formfüllmaschine 5 integriert sein.

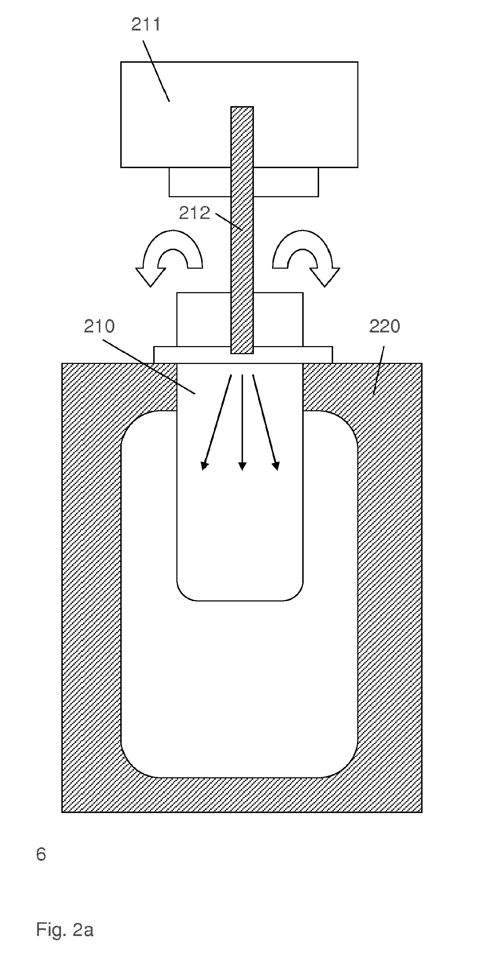

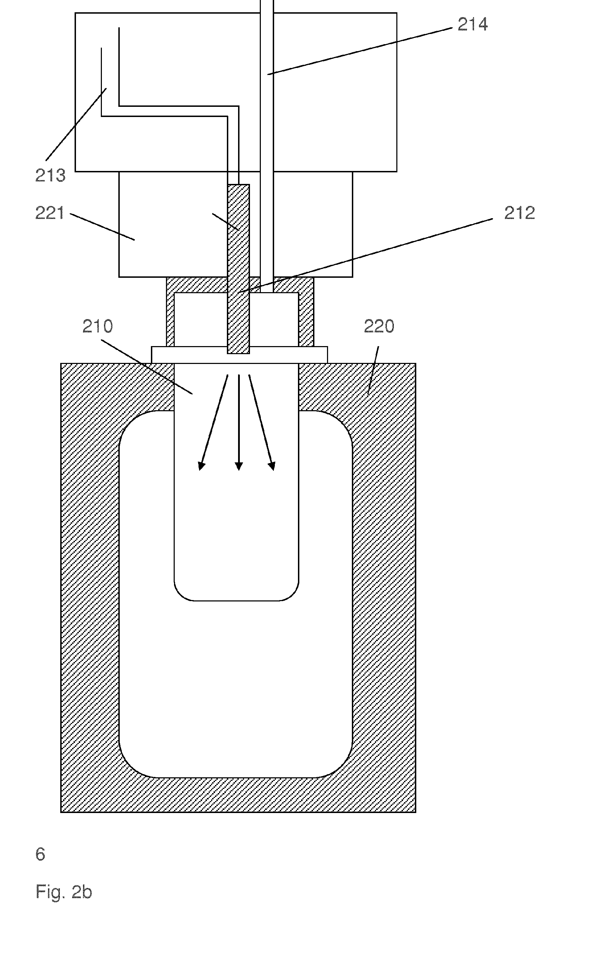

Fig. 2 zeigt eine entsprechende Ausführungsform, bei der die Reinigungseinrichtung in die Formfüllstation 6 der Formfüllmaschine integriert ist. Ein in die Formfüllstation 6 eingebrachter Vorformling 210 wird dazu in einer Form, beispielsweise einer Blasform, 220, in der Formfüllmaschine transportiert. Der Vorformling 210 ist hier noch nicht zum Behälter ausgeformt worden. Das Ausformen kann mittels Druckluft und/oder Verstreckung, aber auch durch Einfüllen von Produkt erfolgen.

In Fig. 2a umfasst die Reinigungseinrichtung ein Reinigungselement, das als in den Vorformling 210 einführbare Lanze 212 ausgebildet ist. Über die Lanze und eine entsprechende mit der Lanze verbundene Leitung, die durch die Lanze 212 und den über dem Vorformling 210 in Position gebrachten Stutzen der Reinigungseinrichtung geführt wird, kann Reinigungsmedium in den Vorformling eingeleitet werden. Dabei kann es sich um ein Gas, beispielsweise ein sterilisierendes Gas, handeln, aber auch um eine Reinigungsflüssigkeit.

An der Lanze können ein oder mehrere Reinigungsventile angeordnet sein. Dabei kann ein Reinigungsventil am unteren, in Richtung Boden des Vorformlings zeigenden Ende der Lanze 212 angeordnet sein. Zusätzlich oder alternativ dazu können weitere Reinigungsventile entlang der Lanze 212 angeordnet sein. Die Reinigungsventile sind geeignet, das Reinigungsmedium in den Behälter zu applizieren.

Um sicherzustellen, dass das Reinigungsmedium vor dem Ausformen vollständig aus dem Vorformling 210 entfernt ist, ist vorgesehen, dass der Vorformling gemäß der in Fig. 2a dargestellten Ausführungsform nach oben hin nicht durch die Reinigungseinrichtung oder andere Bauteile abgeschlossen ist, so dass das Reinigungsmedium aus der Öffnung entweichen kann. Diese Ausführungsform ist insbesondere bei Verwendung von Gasen zur Reinigung des Vorformlings 210 vorteilhaft. Werden Gase verwendet, die eine geringere Dichte aufweisen als die umgebende Atmosphäre bzw. die umgebende Luft, so entweicht dieses Gas von selbst aus dem Vorformling aus der Öffnung. Ist das Gas dichter als Luft, wird es beim Befüllen durch das Produkt verdrängt. Aber auch bei Einsatz einer Flüssigkeit kann beispielsweise durch anschließendes Einblasen von Druckluft die entsprechende Flüssigkeit bzw. das Reinigungsmedium aus dem Vorformling 210 verdrängt werden. Um Verunreinigungen bzw. ständiges Benetzen der Formfüllstationen zu verhindern, kann dazu vorgesehen sein, dass eine Ableitrinne die aus dem Vorformling 210 verdrängte Flüssigkeit ableitet. Die Ableitrinne ist bevorzugt in einem kreisrunden Bereich um die Öffnung der Form 220 ausgebildet.

Hinsichtlich einer Verbesserung des Spül- und Reinigungsergebnisses ist es natürlich denkbar die Ein- und Austragsprozesse wiederholt oder in verschieden Varianten gekoppelt ablaufen zu lassen, wie beispielsweise mehrmaliges Pulsen, abwechselnd Spülen mit gleichen oder verschiedenen Fluiden und oder ein Wechsel von Fluiden und Gasen.

In Fig. 2b ist eine weitere Ausführungsform dargestellt, bei der die Öffnung des Vorformlings 210 verschlossen ist, vorzugsweise luftdicht verschlossen ist. Dieser Verschluss kann beispielsweise über die Lanze 212 realisiert werden, deren Durchmesser in diesem Fall dem Durchmesser der Öffnung des Vorformlings entspricht oder durch einen allgemeinen Behandlungsstutzen 221, der nicht nur die Reinigungseinrichtung sondern beispielsweise auch sämtliche Vorrichtungen zum Ausformen des Vorformlings 210 bis auf die Form 220 umfassen kann. Dazu zählen beispielsweise Reckstangen oder Ventile, die Druckluft oder ein flüssiges Medium zum Ausformen des Vorformlings 210 in den Vorformling 210 einleiten können. Über diesen Behandlungsstutzen (auch Form/Füllblock genannt) kann über eine an einer Lanze 212 angebrachte Düse analog zu Fig. 2 das Reinigungsmedium in den Vorformling eingebracht werden, wobei eine Zuleitung 213 für das Reinigungsmedium vorgesehen ist. Um ein Entleeren des Vorformlings 210 vor dem Ausformen und dem Befüllen sicherzustellen, ist eine Ableitung 214 vorgesehen, die das Reinigungsmedium aus dem Vorformling 210 ableiten kann. Dazu kann auch vorgesehen sein, dass in der Ableitung 214 eine Pumpe vorgesehen ist, die das Reinigungsmedium aus dem Vorformling hinauspumpt. In einer Ausführungsform wird der Spülprozeß dadurch verbessert, dass das Reinigungsmedium unter Druck eingepresst wird und aktiv durch die Ableitung 214 entweicht.

In dieser Ausführungsform ist es besonders vorteilhaft, wenn der Verschluss des Vorformlings 210 auch während des Ausformens und vorzugsweise auch während des Befüllens bestehen bleibt, so dass die gereinigte innere Fläche des Vorformlings 210 bis zum Befüllen nicht kontaminiert werden kann. Um die Zeit zwischen der Reinigung des Vorformlings und dem tatsächlichen Befüllen weiter zu reduzieren, kann auch vorgesehen sein, dass das Ausformen des Vorformlings mit in den Vorformling 210 hineingepumptem Produkt erfolgt, so dass direkt nach der Reinigung das Ausformen zusammen mit dem Einfüllen des Produkts durchgeführt wird.

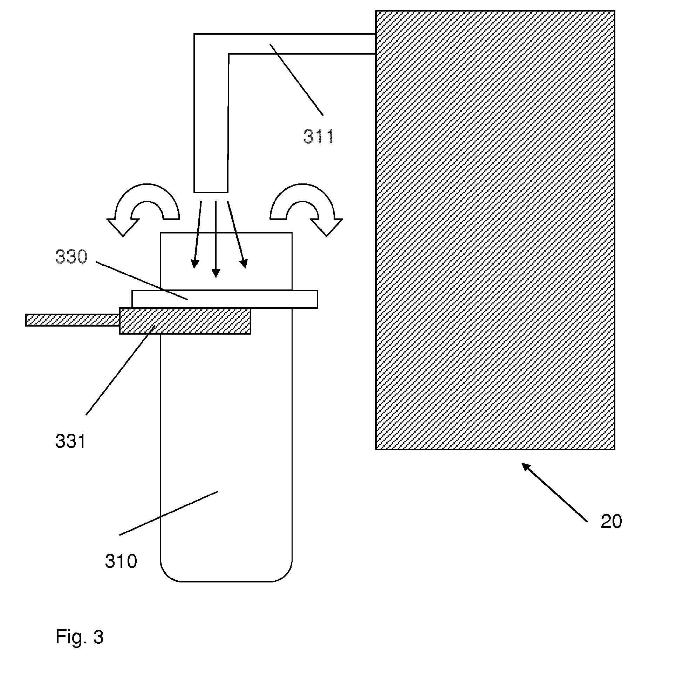

Fig. 3 zeigt eine weitere Ausführungsform, bei der der Vorformling 310 vor Einlauf in die Formfüllanlage gereinigt wird. Dies kann beispielsweise durch eine Reinigungseinrichtung 20, die entlang der Transporteinrichtung angeordnet ist oder mit der Transporteinrichtung 8 selbst mitbewegt wird, entsprechend Fig. 1, erfolgen. Dazu können eine oder mehrere Reinigungsdüsen 311 bereitgestellt werden, die über der Öffnung des Vorformlings 310 positioniert werden können. Besonders vorteilhaft ist hier ein Transport des Vorformlings 310 im Neckhandlingsverfahren, so dass an seinem Kragen 330 eine Klammer 331 angreift und diesen entlang der Transporteinrichtung 8, wie sie in Fig. 1 dargestellt ist, transportiert. Die Düse 311 der Reinigungseinrichtung kann entweder über und damit außerhalb der Öffnung des Vorformlings 310 positioniert werden und das Reinigungsmedium in den Vorformling 310 einlassen oder, insbesondere wenn die Düse mit dem Vorformling mitbewegt wird, in den Vorformling 310 eingeführt werden. Da die Reinigung hier außerhalb der Formfüllanlage stattfindet, ist es nicht notwendig, ein Herausfließen von Reinigungsflüssigkeit bzw. Reinigungsmedium aus der Öffnung des Vorformlings 310 zu verhindern. Es kann sogar in vorteilhafter Weise genutzt werden, um auch die Außenseite des Vorformlings 310 zu reinigen, indem überschüssiges Reinigungsmedium, insbesondere Flüssigkeit, aus der Öffnung des Vorformlings 310 entweicht und an der Außenseite des Vorformlings 310 herunterläuft.

Um den Vorformling 310 danach zuverlässig zu entleeren, kann vorgesehen sein, dass er an der Neckhandlinghalterung weitergeführt und während des Transports gedreht wird. Auch ein Ausblasen mit steriler Luft oder allgemeinem sterilem Gas, wie Stickstoff oder CO₂, kann genutzt werden, um die Innenseite des Vorformlings zu trocknen. Wird auch die äußere Oberfläche des Vorformlings 310 mit flüssigem Reinigungsmedium gereinigt, so ist auch eine Trocknung der Außenseite vorteilhaft, um negative Effekte auf das Ausformen des Vorformlings 310 zum vollständigen Behälter zu vermeiden.

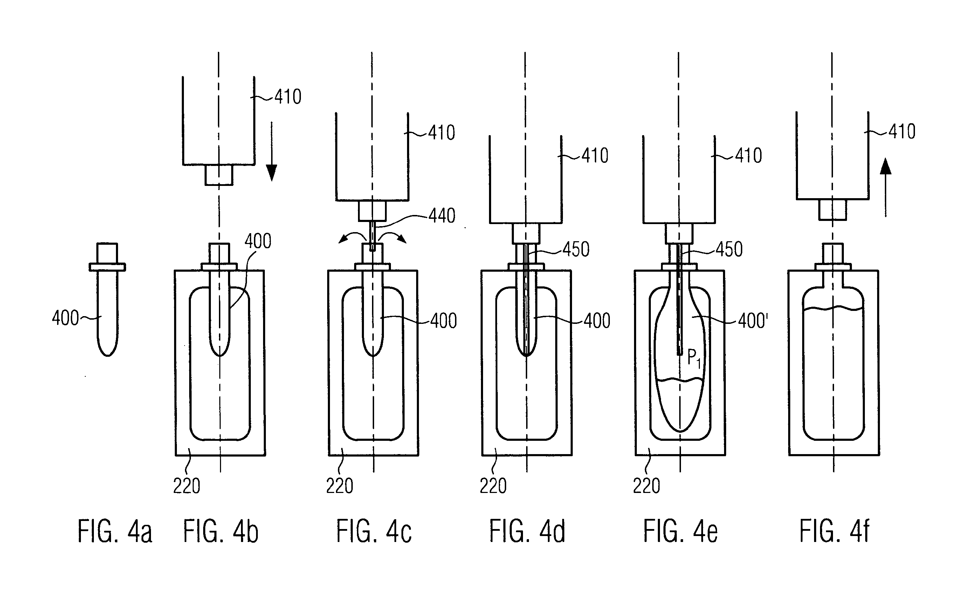

Fig. 4a-4f zeigen eine Ausführungsform eines Verfahrens, bei dem der Vorformling in die Formfüllmaschine, wie sie beispielsweise in Fig. 1 dargestellt ist, eingebracht wird, anschließend luftdicht verschlossen, dann mit einem gasförmigen Reinigungsmedium gereinigt, ausgeformt und anschließend oder zeitgleich befüllt wird, bevor die luftdichte Versiegelung aufgehoben wird.

In Fig. 4a wird der Vorformling beispielsweise mittels Neckhandlingverfahren transportiert. In Fig. 4b hat dann eine Übergabe an die Formfüllmaschine stattgefunden, so dass der Vorformling in eine Form 220 eingesetzt wurde, die um ihn geschlossen wurde. Ein Behandlungsstutzen der Formfüllstation 410, wie er beispielsweise in Fig. 2b beschrieben wurde, wird in Richtung des Vorformlings bzw. dessen Öffnung bewegt.

In Fig. 4c schließt der Behandlungsstutzen die Öffnung des Vorformlings luftdicht ab und mittels einer Düse, die gegebenenfalls auch an einer Lanze 440, bevorzugt an der Reckstange angeordnet sein kann, wird der Vorformling gereinigt. In dieser Ausführungsform wird, wie in Fig. 2b beschrieben, das Reinigungsmedium aus dem Vorformling ausgeleitet, bevor mit dem Ausformen begonnen wird. Dabei wird der luftdichte Verschluss nicht geöffnet. In Fig. 4d kann die Lanze 440 aus dem Vorformling wieder herausgezogen werden und anstelle dieser die Reckstange in den Vorformling 400 eingeführt werden. Ist alternativ eines der oben beschriebenen Reinigungsventile an der Reckstange 450 angeordnet, so erfolgt hier kein Austausch und das Ausformen kann direkt stattfinden. Dazu umfasst die Reckstange ein oder mehrere Formventile, die ein Medium in den Vorformling einleiten können, um das Ausformen zu bewirken. Bei diesem Medium kann es sich beispielsweise um Druckluft, aber auch um eine Flüssigkeit, insbesondere um das in den Behälter einzufüllende Produkt handeln. Es ist mit Hinblick auf die gesamte Dauer des Prozesses vorteilhaft, wenn mehrere Düsen zum Ausbringen eines gasförmigen Reinigungsmediums an der Reckstange angeordnet sind, die beim Einfahren in den Vorformling das Reinigungsmedium einleiten. Unmittelbar danach kann mit dem Ausformen über Verstrecken und Einbringen eines flüssigen oder gasförmigen Mediums begonnen werden. Alternativ zur Reckstange 450 alleine kann ebenso über ein separates Blasventil das Ausformen der Behälter erfolgen. Es ist auch eine geeignete Kombination des Einbringens über die Lanze 440 bzw. Reckstange 450 sowie des separaten Blasventils denkbar.

Der nun im Ausformen befindliche Vorformling 400' ist immer noch luftdicht verschlossen. In Fig. 4e hat der Vorformling 400' fast seine vollständige Größe erhalten und ist damit nahezu vollständig ausgeformt. Bereits zu diesem Zeitpunkt kann, sofern das Ausformen zumindest teilweise durch Einleiten von Produkt erfolgt, ein gewisser Flüssigkeitsspiegel, wie angedeutet, in dem noch nicht vollständig ausgeformten Behälter vorhanden sein. Das von Produkt verdrängte Reinigungsmedium kann durch eine entsprechende Ableitung in dem Behandlungsstutzen definiert entweichen, um so den prozessbedingten Überdruck im Behälter aufrecht zu erhalten . In Fig. 4f ist das Ausformen und das Befüllen vollständig abgeschlossen und der Behandlungsstutzen 410 wird von der Öffnung des Vorformlings entfernt und dabei der luftdichte Verschluss gelöst.

Um den luftdichten Verschluss zu gewährleisten, kann auch vorgesehen sein, dass die Öffnung des Vorformlings durch eine Membran versiegelt wird. Diese Membran kann semipermeabel sein, so dass beispielsweise Reinigungsmedium, wie ein Gas, aus der Membran aus dem Vorformling heraus diffundieren kann. Um den Vorformling zu reinigen, auszuformen oder zu befüllen, kann die Membran an einer Stelle durchstochen und anschließend direkt wieder versiegelt werden. Diese Versiegelung kann auch automatisch durch die Membran selbst, beispielsweise als reversibel verformbare Membran, erfolgen.

Diese Ausführungsform ist besonders dann vorteilhaft, wenn der Vorformling außerhalb der Formfüllmaschine in einer separaten Reinigungseinrichtung gereinigt wird, wie dies beispielsweise in Fig. 3 beschrieben wurde. Während die Reinigung der Innenseite und gegebenenfalls der äußeren Fläche des Vorformlings hier nur unter Verwendung von Gas oder Flüssigkeit beschrieben wurde, ist auch denkbar, dass zur Reinigung Strahlung wie Röntgenstrahlung oder UV-Strahlung verwendet wird oder eine Kombination eines Reinigungsmediums mit Strahlung und/oder Wärmeeinwirkung.

## Patentansprüche

1. Formfüllanlage (1) mit einer Formfüllmaschine (5) zum Formen von Vorformlingen zu Behältern und Füllen der Behälter in einer Form einer oder mehreren Formfüllstationen (6) und mit einem stromaufwärts der Formfüllmaschine (5) angeordneten Heizmodul (7), in dem Vorformlinge aufgeheizt werden können, **dadurch gekennzeichnet, dass** in Transportrichtung der Vorformlinge nach dem Heizmodul (7) eine Reinigungseinrichtung (20) ausgebildet ist, die den Vorformling reinigen kann.

2. Formfüllanlage nach Anspruch 1, **dadurch gekennzeichnet, dass** die Reinigungseinrichtung ein gasförmiges oder flüssiges Reinigungsmedium in den Vorformling einbringen kann.

3. Formfüllanlage nach Anspruch 2, **dadurch gekennzeichnet, dass** das Reinigungsmedium eine sterilisierende Wirkung aufweist.

4. Formfüllanlage nach Anspruch 1 oder 2, **dadurch gekennzeichnet, dass** die Reinigungseinrichtung (20) an einer Transportstrecke vor der Formfüllmaschine angeordnet ist.

5. Formfüllanlage nach Anspruch 1 oder 2, **dadurch gekennzeichnet, dass** die Reinigungseinrichtung in der Formfüllmaschine (5) angeordnet ist und den Vorformling vor dem Ausformen reinigen kann.

6. Formfüllanlage nach Anspruch 5, **dadurch gekennzeichnet, dass** in jeder Formfüllstation (6) ein in den Vorformling einführbares Reinigungselement vorgesehen ist, das ein Reinigungsmedium in den Vorformling einbringen kann.

7. Formfüllanlage nach Anspruch 6, **dadurch gekennzeichnet, dass** ein Behandlungsstutzen (221) der Formfüllstation (6) vorgesehen ist, der das Reinigungselement und ein Formventil umfasst, wobei der Behandlungsstutzen zur Reinigung des Vorformlings in diesen eingeführt werden und den Vorformling luftdicht abschließen kann und wobei durch das Formventil ein Medium in den gereinigten und abgeschlossenen Vorformling zum Ausformen des Behälters eingebracht werden kann.

8. Formfüllanlage nach Anspruch 6, **dadurch gekennzeichnet, dass** das Formventil ausgebildet ist, Produkt in den Behälter einzufüllen.

9. Formfüllanlage nach Anspruch 8, **dadurch gekennzeichnet, dass** das Formventil ausgebildet ist, das Ausformen des Behälters mit dem Produkt als Medium durchzuführen.

10. Verfahren zum Herstellen von Behältern aus Vorformlingen, wobei die Vorformlinge in einem ersten Schritt aufgeheizt werden und in eine Formfüllmaschine (5) transportiert werden, in der sie in einer Form einer Formfüllstation ausgeformt und befüllt werden, **dadurch gekennzeichnet, dass** die Vorformlinge vor dem Ausformen gereinigt werden.

11. Verfahren nach Anspruch 10, **dadurch gekennzeichnet, dass** die Vorformlinge mit einem flüssigen oder einem gasförmigen Medium gereinigt oder auch sterilisiert werden.

12. Verfahren nach Anspruch 10 oder 11, **dadurch gekennzeichnet, dass** die Vorformlinge vor Einlauf in die Formfüllmaschine (5) gereinigt werden.

13. Verfahren nach Anspruch 10 oder 11, **dadurch gekennzeichnet, dass** die Vorformlinge in der Formfüllmaschine (5) gereinigt werden.

14. Verfahren nach Anspruch 13, **dadurch gekennzeichnet, dass** die Vorformlinge während der Reinigung und dem Ausformen kontinuierlich luftdicht verschlossen sind.

15. Verfahren nach Anspruch 14, **dadurch gekennzeichnet, dass** die Vorformlinge befüllt werden, während sie luftdicht verschlossen sind.

16. Verfahren nach Anspruch 14 oder 15, **dadurch gekennzeichnet, dass** das zum Ausformen verwendete Medium das in den Behälter einzufüllende Produkt ist.
